(19) Europäisches Patentamt
European Patent Office
Office européen des brevets

(11) Veröffentlichungsnummer: **0 189 586 B1**

# (12) EUROPÄISCHE PATENTSCHRIFT

(45) Veröffentlichungstag der Patentschrift: **24.05.89**

(51) Int. Cl.⁴: **C 07 D 317/32**, A 61 K 9/28 // C07D317/20, C07D319/06

(21) Anmeldenummer: **85116437.6**

(22) Anmeldetag: **21.12.85**

(54) **Verfahren zur Herstellung von Salzen der 1,3-Dioxolan-4-carbonsäuren.**

(30) Priorität: **29.12.84 DE 3447783**

(43) Veröffentlichungstag der Anmeldung: **06.08.86 Patentblatt 86/32**

(45) Bekanntmachung des Hinweises auf die Patenterteilung: **24.05.89 Patentblatt 89/21**

(84) Benannte Vertragsstaaten: **DE**

(56) Entgegenhaltungen:
**DE-A- 2 045 090**
**DE-A- 2 404 072**
**DE-A- 2 903 388**
**US-A- 3 595 909**

**CHEMISCHE BERICHTE, Band 109, Nr. 11, 1976, Seiten 3707-3727, Verlag Chemie GmbH, Weinheim; K. HEYNS et al.: "Über selektive katalytische Oxidationen, XXXVII. Platin-Katalyse als Hydridmechanismus : Die katalytische Oxidation stickstoff- und sauerstoffhaltiger Heterocyclen"**
**ANGEWANDTE CHEMIE, Band 69, Nr. 18/19, 25. September 1957, Seiten 600-608- Verlag Chemie GmbH, Weinheim; K. HEYNS et al.: "Neuere Methoden der präparativen organischen Chemie II. 8. Selektive katalytische Oxydationen mit Edelmetallkatalysatoren"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Möller, Hinrich, Dr., Schumannstrasse 11, D-4019 Monheim (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 189 586 B1

ACTORUM AG

**Beschreibung**

Gegenstand der Erfindung ist ein neues Verfahren zur Herstellung von Salzen der 1,3-Dioxolan-4-carbonsäuren.

1,3-Dioxolan-4-carbonsäuren sind als Ausgangsprodukte zur Herstellung von Glycerinsäure und Glycerinsäureestern geeignet. Wegen ihrer schwierigen Zugänglichkeit werden sie selbst aber bisher durch Acetalisierung oder Ketalisierung von Glycerinsäureestern z.B. gemäss DE-A-20 45 090 gewonnen. Glycerinsäure ist durch direkte Oxidation von Glycerin mit Salpetersäure auf umständliche und wenig ergiebige Weise zugänglich.

Es wurde gefunden, dass Salze von 1,3-Dioxolan-4-carbonsäure gute feuchtigkeitsbindende Eigenschaften aufweisen und sich daher als Feuchthaltemittel für technische Zwecke und als Hautfeuchthaltemittel in kosmetischen Zubereitungen eignen. Es bestand daher ein Bedürfnis nach einem ergiebigeren Herstellverfahren für 1,3-Dioxolan-4-carbonsäuren.

Die Acetalisierung oder Ketalisierung von Glycerin mit niederen Aldehyden oder Ketonen führt zu einem Gemisch isomerer cyclischer Acetale, bestehend aus 4-Hydroxymethyl-1,3-dioxolanen und 5-Hydroxy-1,3-dioxanen, welche wegen eng beieinanderliegender Siedepunkte nur schwer voneinander zu trennen sind (vgl. J. Am. Chem. Soc. Vol. 50, (1928), S. 2242–2249 und 3120–3127).

Während bei der Bildung der cyclischen Ketale das 5-Hydroxy-1,3-Dioxan nur in untergeordneten Mengen entsteht, wird bei der Umsetzung von Glycerin mit Aldehyden, wie z.B. Formaldehyd und Acetaldehyd, das 5-Hydroxy-1,3-dioxan in Mengen bis zu 40 Gew.-% des Isomerengemisches gebildet. Die Konzentration des 5-Hydroxy-1,3-dioxans kann durch allmähliche spontane Umlagerung des 4-Hydroxymethyl-1,3-dioxolans, besonders in Gegenwart von Säurespuren, noch weiter zunehmen. Technisches Glycerinformal kann daher bis zu 80 Gew.-% des 5-Hydroxy-1,3-dioxans enthalten.

Es wurde gefunden, dass die Herstellung von Salzen der 1,3-Dioxolan-4-carbonsäuren der allgemeinen Formel (I)

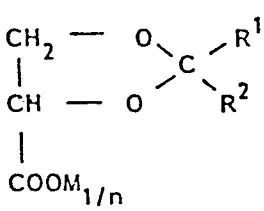

(I)

in der $R^1$ und $R^2$ Wasserstoff oder eine Alkylgruppe mit 1–4 C-Atomen und M ein Alkali-, Erdalkali-, Ammonium-, Mono-, Di- oder Trialkanolammonium mit 2 oder 3 C-Atomen in der Alkanolgruppe und n dessen Wertigkeit bedeutet, aus dem Isomerengemisch von 4-Hydroxymethyl-1,3-dioxolan und 5-Hydroxy-1,3-dioxan, welches bei der Umsetzung von Glycerin mit einem Aldehyd oder Keton der allgemeinen Formel $R^1R^2CO$, in der $R^1$ und $R^2$ die vorgenannte Bedeutung haben, erhalten wird, dadurch leicht möglich ist, dass man

– das Isomerengemisch in Gegenwart eines Katalysators aus der Gruppe der Platinmetalle und einer Base bei einem pH-Wert von 7–14 mit Sauerstoff oder Luft oxidiert und

– aus dem Reaktionsgemisch das Salz der 1,3-Dioxolan-4-carbonsäure der Formel I isoliert.

Unter den erfindungsgemässen Oxidationsbedingungen wird nur das 4-Hydroxymethyl-1,3-dioxan oxidiert, so dass aus dem Reaktionsgemisch auch das 5-Hydroxy-1,3-dioxan der allgemeinen Formel II

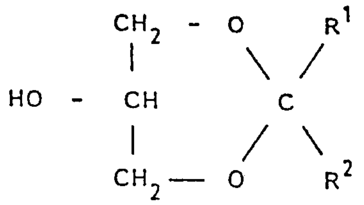

(II)

in der $R^1$ und $R^2$ die vorgenannte Bedeutung haben, leicht isoliert werden kann.

Das erfindungsgemässe Verfahren ist von besonderem Wert für die Herstellung der 1,3-Dioxolan-4-carbonsäuren der allgemeinen Formel (I), in der $R^1$ Wasserstoff und $R^2$ eine Alkylgruppe mit 1–4 C-Atomen darstellt.

Die Oxidation wird in wässrigem Medium bei einem pH-Wert von 7–14, d.h. im alkalischen Bereich durchgeführt. Als besonders geeignete Basen haben sich z.B. Natrium- und Kaliumhydroxid, Natrium- und Kaliumcarbonat, Natrium- und Kaliumhydrogencarbonat, Calciumhydroxid und Bariumhydroxid bewährt. Geeignet sind aber auch Ammoniak, Mono-, Di- und Trialkanolamine mit 2–4 C-Atomen in der Alkanolgruppe. Die Base wird bevorzugt in einer Menge von 1–1,5 Mol pro Mol 4-Hydroxymethyl-1,3-dioxolan dem Isomerengemisch zugegeben.

Als Katalysator aus der Gruppe der Platinmetalle wird bevorzugt Palladium oder Platin verwendet. Besonders hohe Ausbeuten werden erhalten, wenn eine mit Palladium beaufschlagte Aktivkohle als Katalysator eingesetzt wird. Als Oxidationsmittel dient Sauerstoff, entweder in Form von reinem Sauerstoff oder in Form von Luft. Die Oxidation kann bei Normaldruck durchgeführt werden. Bevorzugt wird jedoch ein leichter Überdruck angewendet. Besonders bei Verwendung von Luft wird durch Anwendung eines Überdrucks bis zu 100 bar eine Beschleunigung der Reaktion erzielt. Die Temperatur bei der Oxidation kann zwischen 20 und 100 °C liegen, bevorzugt wird eine Temperatur von 50–100 °C gewählt. Der Fortschritt der Reaktion kann durch Titration der entstehenden 1,3-Dioxolan-4-carbonsäure kontrolliert werden. Bei Verwendung von Luft als Oxidationsmittel ist die Oxidation bei 50–100 °C unter Normaldruck innerhalb 2 bis 6 Stunden beendet. Bei Anwendung von Sauerstoff oder von Überdruck kann die Reaktionszeit auf 1–2 Stunden verringert werden.

Nach beendeter Oxidation wird der Katalysator, z.B. durch Filtration oder Sedimentation entfernt und der pH-Wert auf einen Wert von 8–9 eingestellt, um sicherzustellen, dass die gebildete 1,3-Dioxolan-4-carbonsäure in Salzform vorliegt. Aus dem so erhaltenen Reaktionsgemisch kann das nicht oxidierte 5-Hydroxy-1,3-dioxan leicht durch Extraktion mit einem Lösungsmittel abgetrennt werden, in welchem das 5-Hydroxy-1,3-dioxan löslich, das Salz der 1,3-Dioxolan-4-carbonsäure aber nicht löslich ist. Solche Lösungsmittel sind z.B. chlorierte Kohlenwasserstoffe wie Methylenchlorid, Chloroform, Tetrachlorkohlenstoff, Perchlorethylen, flüssige Kohlenwasserstoffe und andere wasserunlösliche flüssige Lösungsmittel, in welchen sich die Salze der 1,3-Dioxolan-4-carbonsäure nicht lösen.

Besonders vorteilhaft ist die Extraktion nach Entfernung des Wassers, z.B. durch Abdestillation, aus dem weitgehend wasserfreien Rückstand. Es können dann auch wasserlösliche Lösungsmittel, insbesondere flüssige Ether wie z.B. Dioxan, Ethylenglykoldimethylether oder Methyl-tert.-butylether für die Extraktion eingesetzt werden. Die durch Extraktion gereinigten Salze der 1,3-Dioxolan-4-carbonsäure können durch Umkristallisieren aus niederen Alkoholen, z.B. aus Methanol weiter gereinigt werden.

Aus den Lösungsmittel-Extrakten können die 5-Hydroxy-1,3-Dioxane der allgemeinen Formel II durch Entfernung des Lösungsmittels und Destillation isoliert und gereinigt werden. Die 5-Hydroxy-1,3-dioxane selbst sind als Lösungsmittel für polare organische Substanzen geeignet.

Die Salze der 1,3-Dioxolan-4-carbonsäuren der allgemeinen Formel I weisen ein hohes Feuchtigkeitsbindevermögen auf. Sie eignen sich daher als Feuchthaltemittel sowohl für technische als auch für kosmetische Zwecke. Besonders interessant ist die gute hautfeuchthaltende Wirkung der Verbindungen.

Beispiele

1. Herstellung von 1,3-Dioxolan-4-carbonsäure, Natriumsalz

40 g des aus Glycerin und Paraformaldehyd herstellbaren Glycerinformals (385 mMol), (bestehend aus 25 Gew.-% 4-Hydroxymethyl-1,3-dioxolan und 75 Gew.-% 5-Hydroxy-1,3-dioxan) und 3,8 g Natriumhydroxid (96 mMol) wurden in 360 ml Wasser gelöst. Die Lösung wurde in ein vertikales Rohr mit 40 mm Durchmesser mit einem Sinterglasboden (Chromatographierohr), welches durch einen Flüssigkeitsmantel beheizbar war, gegeben und auf 70 °C erhitzt. Nach Zugabe von 4 g einer mit 5 Gew.-% Palladium beaufschlagten Aktivkohle wurde Luft mit einer Strömungsgeschwindigkeit von 0,8 l/min 3 Stunden lang bei 70 °C durch den Sinterglasboden geleitet.

Nach Filtration wurde der auf 6,5 abgesunkene pH-Wert durch Zugabe von Natriumhydroxidlösung auf 8,5 angehoben und das Wasser unter vermindertem Druck (Wasserstrahlvakuum) abdestilliert. Der Rückstand wurde dreimal mit je 50 ml Ethylenglykoldimethylether ausgewaschen. Der Rückstand ergab nach dem Trocknen 11 g (82% d.Th.) 1,3-Dioxolan-4-carbonsäure-Na-Salz. Nach Umlösen aus Methanol lag der Schmelzpunkt bei 230–234 °C.

Aus dem Ethylenglykoldimethylether-Extrakt wurde nach Abdestillieren des Lösungsmittels und Destillation des Rückstandes 27,1 g (90% d.Th.) reines 5-Hydroxy-1,3-dioxolan mit einem Siedepunkt von 192 °C und einem Brechungsindex $n^{20}$ = 1,4529 erhalten.

2. Herstellung von 2,2-Dimethyl-1,3-dioxolan-4-carbonsäure, Natriumsalz

40,7 g des aus Glycerin und Aceton herstellbaren Glycerin-Ketals (0,31 Mol) mit einem Gehalt von 97,5 Gew.-% 2,2-Dimethyl-4-hydroxymethyl-1,3-dioxolan und 12,2 g Natriumhydroxid (0,3 Mol) wurden in 366 ml Wasser gelöst. Die Lösung wurde in Gegenwart von 4 g einer mit 5 Gew.-% Pd beaufschlagten Aktivkohle unter den in Beispiel 1 genannten Bedingungen mit Luft oxidiert.

Nach Abfiltration des Katalysators wurde die Lösung, die noch einen pH-Wert von 9,5 aufwies, durch Zugabe von Schwefelsäure auf einen pH-Wert von 8,5 eingestellt und das Wasser unter vermindertem Druck (Wasserstrahlvakuum) abdestilliert. Der Rückstand wurde mit Methyl-tert.-butylether heiss extrahiert. Dabei wurden 1,1 g des Produktes herausgelöst. Der Rückstand wurde in Methanol gelöst, von ungelösten Anteilen abfiltriert und die Lösung zur Trockene eingeengt. Als Rückstand wurden 45 g (92% d.Th., bezogen auf umgesetztes Produkt) des reinen Natriumsalzes der 2,2-Dimethyl-1,3-dioxolan-4-carbonsäure erhalten. Das rohe Produkt zeigte einen Schmelzpunkt von 243–245 °C, nach Umlösen aus Methanol lag der Schmelzpunkt bei 249–251 °C.

2a. Oxidation unter Druck

40,7 g des Glycerinketals (0,31 Mol) mit einem Gehalt von 97,5 Gew.-% 2,2-Dimethyl-4-hydroxymethyl-1,3-dioxolan und 12,2 g Natriumhydroxid (0,3 Mol) wurden in 366 ml Wasser gelöst. Die Lösung wurde in Gegenwart von 4 g einer mit 5 Gew.-% Pd beaufschlagten Aktivkohle in einem Nickelschüttelautoklaven bei 25 bar Luftdruck innerhalb von 2 Stunden unter Schütteln auf 80 °C erwärmt. Dabei stieg der Druck kurzzeitig auf 30 bar an, fiel dann wieder auf 25 bar ab und wurde nach einer weiteren Stunde auf 63 bar erhöht. Nach 2 Stunden blieb der Druck konstant bei 53 bar. Der Autoklav wurde entspannt und das Reaktionsgemisch analog Beispiel 2 aufgearbeitet. Das 2,2-Dimethyl-1,3-dioxolan-4-carbonsäure-Na-Salz wurde in einer Ausbeute von 86% d.Th. mit einem Schmelzpunkt von 243–246 °C isoliert.

3. Herstellung von 2-Ethyl-2-methyl-1,3-dioxolan-4-carbonsäure, Natriumsalz

45,2 g des aus Glycerin und Methylethylketon herstellbaren, rohen 2-Ethyl-2-methyl-4-hydroxymethyl-1,3-dioxolans (0,31 Mol) wurden unter den Bedingungen des Beispiels 2 in Gegenwart von 4 g einer mit 5 Gew.-% Palladium beaufschlagten Aktivkohle mit Luft oxidiert. Nach Aufarbeitung gemäss Beispiel 2 wurde das 2-Ethyl-2-methyl -1,3-dioxolan -4-carbonsäure-Natriumsalz in einer Ausbeute von 85% d.Th. mit einem Schmelzpunkt von 273–276 °C erhalten.

## Patentansprüche

1. Verfahren zur Herstellung von Salzen der 1,3-Dioxolan-4-carbonsäure der allgemeinen Formel I

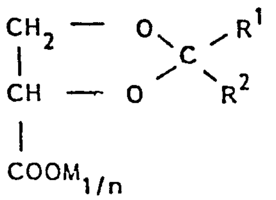

(I)

in der $R^1$ und $R^2$ Wasserstoff oder eine Alkylgruppe mit 1–4 C-Atomen und M eine Alkali, Erdalkali-, Ammonium-, Mono-, Di- oder Trialkanolammoniumion mit 2 oder 3 C-Atomen in der Alkanolgruppe und n dessen Wertigkeit bedeutet, aus dem Isomerengemisch von 4-Hydroxymethyl-1,3-dioxolan und 5-Hydroxy-1,3-dioxan, welches bei der Umsetzung von Glycerin mit einem Aldehyd oder Keton der allgemeinen Formel $R^1R^2CO$, in der $R^1$ und $R^2$ die vorgenannte Bedeutung haben, entsteht, dadurch gekennzeichnet, dass man

– das Isomerengemisch in Gegenwart eines Katalysators aus der Gruppe der Platinmetalle und einer Base bei einem pH-Wert von 7–14 mit Sauerstoff oder Luft oxidiert und

– aus dem Reaktionsgemisch das Salz der 1,3-Dioxolan-4-carbonsäure der Formel I isoliert.

2. Verfahren nach Anspruch 1, dadurch gekennzeichnet, dass in der allgemeinen Formel I $R^1$ Wasserstoff und $R^2$ eine Alkylgruppe mit 1–4 C-Atomen darstellt.

3. Verfahren nach Anspruch 1, dadurch gekennzeichnet, dass man als Base Natrium- oder Kaliumhydroxid, Natrium- oder Kaliumcarbonat, Natrium- oder Kaliumhydrogencarbonat, Calcium- oder Bariumhydroxid verwendet.

4. Verfahren nach Anspruch 1–3, dadurch gekennzeichnet, dass man die Oxidation mit Sauerstoff oder Luft bei einem Druck von 1–100 bar und bei einer Temperatur von 50–100 °C durchführt.

5. Verfahren nach Anspruch 1–4, dadurch gekennzeichnet, dass man nach der Oxidation das 5-Hydroxy-1,3-dioxan aus dem Reaktionsgemisch durch Extraktion mit einem Lösungsmittel abtrennt.

## Revendications

1. Procédé pour la fabrication de sels d'acide 1,3-dioxolane-4-carboxylique de formule générale I,

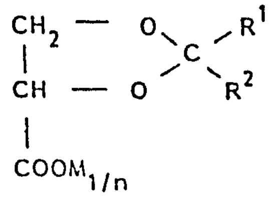

(I)

dans laquelle $R^1$ et $R^2$ représentent de l'hydrogène ou un groupe alkyle de 1 à 4 atomes de carbone et M représente un ion de métal alcalin, alcalino-terreux, ammonium, mono-, di- ou tri-alkanolammonium comportant 2 ou 3 atomes de carbone dans le groupement alkanol et n signifie sa valence, à partir d'un mélange d'isomères de 4-hydroxyméthyl-1,3-dioxolane et de 5-hydroxy-1,3-dioxane, qui se forme en faisant réagir la glycérine avec un aldehyde ou une cétone de formule générale $R^1R^2CO$, dans laquelle $R^1$ et $R^2$ ont la signification mentionnée plus haut, caractérisé en ce qu'on effectue une oxydation avec l'oxygène ou avec l'air du mélange d'isomères en présence d'un catalyseur du groupe des métaux de platine et d'une base, à une valeur de pH comprise entre 7 et 14, et en ce qu'on sépare le sel de l'acide 1,3-dioxolane-4-carboxylique de formule I du mélange d'isomères.

2. Procédé selon la revendication 1, caractérisé en ce que dans la formule générale I, $R^1$ représente l'hydrogène et $R^2$ un groupe alkyle comportant 1 à 4 atomes de carbone.

3. Procédé selon la revendication 1, caractérisé en ce qu'on emploie comme base l'hydroxyde de sodium ou de potassium, le carbonate de sodium ou de potassium, le bicarbonate de sodium ou de potassium, l'hydroxyde de calcium ou de baryum.

4. Procédé selon les revendications 1–3, caractérisé en ce qu'on effectue l'oxydation avec l'oxygène ou avec l'air sous une pression de 1–100 bar et à une température de 50 à 100 °C.

5. Procédé selon les revendications 1–4, caractérisé en ce qu'on sépare le 5-hydroxy-1,3-dioxane du mélange réactionnel après l'oxydation par extraction avec un solvant.

## Claims

1. A process for the production of salts of 1,3-dioxolane-4-carboxylic acid corresponding to the following general formula

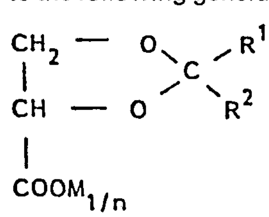

(I)

in which $R^1$ and $R^2$ represent hydrogen or a $C_1$–$C_4$ alkyl group and M is an alkali, alkaline earth, ammonium, mono-, di- or trialkanolammonium ion containing 2 or 3 carbon atoms in the alkanol group and n is its valency, from the isomer mixture of 4-hydroxymethyl-1,3-dioxolane and 5-hydroxy-1,3-dioxane which is obtained in the reaction of glycerin with an aldehyde or ketone corresponding to the general formula $R^1R^2CO$, in which

$R^1$ and $R^2$ are as defined above, characterized in that

– the isomer mixture is oxidized with oxygen or air in the presence of a catalyst from the group of platinum metals and a base at a pH-value of from 7 to 14 and

– the salt of the 1,3-dioxolane-4-carboxylic acid of formula I is isolated from the reaction mixture.

2. A process as claimed in Claim 1, characterized in that, in general formula I, $R^1$ is hydrogen and $R^2$ is a $C_1$–$C_4$ alkyl group.

3. A process as claimed in Claim 1, characterized in that sodium or potassium hydroxide, sodium or potassium carbonate, sodium or potassium hydrogen carbonate, calcium or barium hydroxide is used as the base.

4. A process as claimed in Claim 1 to 3, characterized in that oxidation is carried out with oxygen or air under a pressure of from 1 to 100 bar and at a temperature of from 50 to 100 °C.

5. A process as claimed in Claims 1 to 4, characterized in that, on completion of oxidation, the 5-hydroxy-1,3-dioxane is removed from the reaction mixture by extraction with a solvent.